# EUROPÄISCHE PATENTSCHRIFT

(11) **EP 2 072 073 B1**
(45) Veröffentlichungstag und Bekanntmachung des Hinweises auf die Patenterteilung: **28.12.2011**
(21) Anmeldenummer: 08020283.1
(22) Anmeldetag: 21.11.2008
(51) Int. Cl.: A61M 15/08

(54) **Austragvorrichtung zur nasalen Anwendung**
Discharge device for nasal use
Dispositif de décharge destiné à l'application nasale

(30) Priorität: 20.12.2007 DE 102007063213
(43) Veröffentlichungstag der Anmeldung: 24.06.2009
(73) Patentinhaber: Ing. Erich Pfeiffer GmbH, 78315 Radolfzell (DE)
(72) Erfinder: Stadelhofer, Peter, 78224 Singen (DE)
(74) Vertreter: Patentanwälte Ruff, Wilhelm, Beier, Dauster & Partner

(56) Entgegenhaltungen:
- WO-A-01/78818
- WO-A-2006/097753
- DE-A1- 4 037 783
- FR-A- 517 936
- FR-A- 2 764 807
- US-A- 2 195 554

## Beschreibung

### Anwendungsgebiet und Stand der Technik

Die Erfindung betrifft eine Austragvorrichtung zur nasalen Applikation von Medikamenten.

Solche Austragvorrichtungen finden zumeist in Form von Nasensprays Verwendung, die bei Erkältungen Linderung bringen sollen. Ein besonders üblicher Typ solche Austragvorrichtungen weist eine Nasenolive auf, die sich von einem Grundkörper in Richtung von dessen Haupterstreckungsrichtung erstreckt und zu einem Austrag in Richtung dieser Haupterstreckungsrichtung ausgebildet sind. Der Nachteil an diesem Typ Austragvorrichtung ist, dass die intuitive Anwendung durch den Benutzer zumeist dergestalt ist, dass der Benutzer die Austragvorrichtung und damit die Nasenolive bei der Verwendung derart hält, dass der Austrag nahezu vertikal erfolgt. Dies ist jedoch in medizinischer Hinsicht nicht ideal, da die zu erreichende Nasenhöhle abweichend von einem weit verbreiteten Irrglauben nicht oberhalb sondern hinter den Nasenlöchern angeordnet ist. Die unter medizinischen Gesichtspunkten vorteilhafte nahezu waagerechte Austragrichtung wird durch den beschriebenen Typ von Austragvorrichtungen zumeist nicht erreicht.

Zwar sind aus dem Stand der Technik, beispielsweise aus der DE 195 27 943 A1, auch Sprühflaschen für Ohrentropfen bekannt, bei denen ein Mündungsteil gegenüber einer das entsprechende Medium speichernden Flasche und der Haupterstreckungsrichtung schwenkbar ist, diese gestatten jedoch eine variable Einstellung der Schwenkrichtung, die es einem Benutzer sehr einfach machen, wieder in der gewohnten nachteiligen Art und Weise einen weitgehend vertikal ausgerichteten Sprühstrahl zu erzeugen.

Um die richtige Austragrichtung zu gewährleisten, schlägt die US-Patentanmeldung US 2006/0137683 A1 vor, die Austragöffnung an einer ansonsten weitgehend konventionell gearteten Nasenolive derart anzuordnen, dass der Sprühstrahl nicht in Richtung der Haupterstreckungsrichtung der Nasenolive, sondern um etwa 90° abgewinkelt ausgerichtet ist. Die Nasenolive wird demnach in der den meisten Benutzern gängigen Art und Weise vertikal in das Nasenloch eingeschoben und führt dann zu einem Sprühstrahl, der in etwa horizontal ausgerichtet ist.

Nachteilig an dieser Ausgestaltung ist allerdings, dass für den Benutzer schwer einzuschätzen ist, wie weit die Nasenolive in das Nasenloch einzuführen ist, bevor das Medium versprüht werden kann. Wird das Medium vor Erreichen der Idealposition der Nasenolive ausgetragen, so schlägt es sich im Nasenloch nieder, ohne die Nasenhöhle zu erreichen. Eine weitese Nasenspray-Vorrichtung wird in der veröffentlichten Patentanweldung US 219 5554 A beschrieben.

### Aufgabe und Lösung

Aufgabe der Erfindung ist es, eine Austragvorrichtung zur nasalen Applikation von Medikamenten zur Verfügung zu stellen, die intuitiv durch den Benutzer richtig gehandhabt wird und die ein in medizinischer Hinsicht vorteilhaftes Sprühverhalten aufweist.

Erfindungsgemäß wird dies durch eine Austragvorrichtung zur nasalen Applikation von Medikamenten gemäβ Anspruch 1 erreicht, die einen Hauptkörper aufweist, der eine bei Benutzung dem Gesicht des Benutzers zugewandte und vertikal ausgerichtete Anlagefläche aufweist, und die weiterhin eine ortsfest zum Hauptkörper angeordnete Nasenolive zur Einführung in ein Nasenloch des Benutzers aufweist, wobei die Nasenolive an einem oberen Randbereich der Anlagefläche vorgesehen ist, sich über die Anlagefläche erhebt und eine Austragöffnung aufweist.

Im Zusammenhang mit dieser Erfindung sind Ausrichtungs- und Richtungsangaben wie beispielsweise "oben", "und" und "vertikal" sowie "horizontal" auf eine Benutzungssituation bezogen, in der der Benutzer aufrechten Kopfes die Austragvorrichtung in ihrer bestimmungsgemäßen Ausrichtung hält. Eine abweichende, beispielsweise liegende, Haltung des Benutzers oder eine Nicht-Benutzungssituation der Austragvorrichtung, beispielsweise im gelagerten Zustand, führen dementsprechend zu einer entsprechenden Veränderung hinsichtlich der im folgenden genutzten Angaben bezüglich der Richtung und Ausrichtung.

Die erfindungsgemäße Austragvorrichtung weist einen Hauptkörper auf, in dem vorzugsweise alle zur Speicherung und Förderung des Mediums erforderlichen Bauteile enthalten sind, insbesondere also ein Medienspeicher, eine Förder- oder Pumpeinrichtung sowie Medienleitungen, die einen Leitungsweg für das Medium vom Medienspeicher bis zur Austragöffnung bilden. An einer Seite weist die Austragvorrichtung eine Anlagefläche auf, deren Zweck es ist, die korrekte Ausrichtung der Austragvorrichtung einem Benutzer intuitiv richtig machen zu lassen und eine medizinisch unerwünschte Ausrichtung unmöglich oder schwer möglich zu machen. Diese Anlagefläche ist bei der bestimmungsgemäßen Benutzung der unteren Gesichtshälfte des Benutzers zugewandt und befindet sich unmittelbar vor dem Mund und/oder dem Kinn des Benutzers oder liegt an diesen Körperteilen unmittelbar an. Bei dieser Haltung ist die Nasenolive, die sich über diese Anlagefläche erhebt und dadurch in der beschriebenen Lage auf den Benutzer zu weist, in ein Nasenloch des Benutzers eingeschoben. Die Nasenolive, die an einem oberen Randbereich der Anlagefläche vorgesehen ist, erstreckt sich also zumindest mit einer horizontalen Richtungskomponente bis in ein Nasenloch des Benutzers hinein. Bei einer besonderen Ausgestaltung sind zwei Nasenoliven an der Anlagefläche vorgesehen, die den zeitgleichen Austrag des Mediums in beide Nasenlöcher des Benutzers zulassen.

Die Ortsfestigkeit der Nasenolive relativ zum Hauptkörper führt zur besonders angenehmen Verwendung, da die den Hauptkörper umgreifende Hand des Benutzers unschwer relativ zur Nase des Benutzers ortsfest gehalten werden kann und dies dadurch auch zu einer relativ zur Nase ortsfesten Lage der Nasenolive führt. Eine Betätigung der Austragvorrichtung bringt keine als unangenehm empfundene Verschiebung der Nasenolive in die Nase hinein oder aus der Nase heraus mit sich.

Eine davon abweichende nachteilige Ausrichtung der Austragvorrichtung, insbesondere eine Ausrichtung, bei der die Nasenolive eine in etwa vertikale Ausrichtung einnimmt, ist mit der erfindungsgemäßen Austragvorrichtung nur schwer zu erreichen, da ein Anstoßen der Anlagefläche am Mund und/oder Kinn dies verhindert. Zwar könnte die Austragvorrichtung in ihrer Gesamtheit um die Haupterstreckungsrichtung der Nasenolive um 90° verschwenkt werden, aufgrund der dann asymmetrischen Haltung, bei der die Anlagefläche entweder rechtsseitig oder linksseitig der Nase vorgesehen ist, ist für den Benutzer jedoch unmittelbar ersichtlich, dass dies nicht die bestimmungsgemäße Haltung sein kann. Die Anlagefläche selbst muss nicht vollständig eben sein, ist jedoch in der vertikalen vorzugsweise allenfalls leicht gekrümmt. Vorzugsweise ist die Fläche zumindest abschnittsweise vollständig eben. Sie braucht keine große Breite zu haben, da es nur darauf ankommt, dass sie am Mund und/oder am Kinn des Benutzers zum Anliegen kommt. Eine Breite von einigen Millimetern, vorzugsweise von 10 mm oder etwas mehr, wird als bevorzugt angesehen. Die Höhe der Anlagefläche beträgt vorzugsweise mindestens 40 mm, insbesondere vorzugsweise mindestens 60 mm. Hierdurch ist ein sicheres Anliegen am Mund und gegebenenfalls am Kinn gewährleistet.

Die Nasenolive erhebt sich vorzugsweise um mindestens 10 mm über die Anlagefläche. Diese Distanz bezieht sich auf ein distales Ende der Nasenolive sowie auf eine auf der Anlagefläche oder einer mit dieser fluchtenden gedachten Fläche stehende Flächennormale. Die Länge von 10 mm gewährleistet ein sicheres Eintauchen der Nasenolive in das Nasenloch in horizontaler Richtung. Besonders bevorzugt sind Ausgestaltungen mit einer Erhebung der Nasenolive von mindestens 15mm, insbesondere von mindestens 20mm.

Bei einer bevorzugten Ausgestaltung der Erfindung erstreckt sich die Haupterstreckungsrichtung des Hauptkörpers parallel zur vertikal ausgerichteten Anlagefläche. Die Haupterstreckungsrichtung des Hauptkörpers entspricht der Raumrichtung, in der der Hauptkörper die größte Erstreckung aufweist. Bei einer bevorzugten etwa zylindrischen Formgebung des Hauptkörpers entspricht die Haupterstreckungsrichtung der Zylinderachse. Vorzugsweise verläuft die Haupterstreckung des Hauptkörpers weitgehend vertikal oder in einem Winkel von weniger als 15° zur Vertikalen. Der Hauptkörper weist dadurch eine insgesamt schlanke Gestalt auf, die eine vertikale Ausrichtung des Hauptkörpers während der Betätigung bereits nahe legt. Die schlanke Gestalt ist darüber hinaus für die Handhabbarkeit besonders von Vorteil.

Eine Haupterstreckungsrichtung der Nasenolive schließt mit einer Vertikalen vorzugsweise einen Winkel zwischen 45° und 90° ein, insbesondere zwischen 60° und 90°. Insbesondere von Vorteil ist es, wenn die Haupterstreckungsrichtung nahezu im rechten Winkel zur Vertikalen, also horizontal, verläuft. Als Haupterstreckungsrichtung der Nasenolive wird jene Richtung angesehen, in die die Nasenolive zur Einführung in das Nasenloch bewegt wird. Diese Haupterstreckungsrichtung entspricht dabei vorzugsweise einer Richtung, die zu einer gedachten Linie parallel ist, welche durch zwei Punkte definiert wird, einerseits dem distalen Ende der Nasenolive und andererseits dem Mittelpunkt der Querschnittsfläche der Nasenolive in der Ebene der Anlagefläche. Diese Gestaltung gewährleistet, dass die Nasenolive besonders gut in das Nasenloch einführbar ist. Aufgrund des großen Winkels, den die Nasenolive mit der Vertikalen einschließt, ist die Einführungsbewegurig nah an einer horizontalen Bewegung.

Bei einer bevorzugten Ausgestaltung ist die Nasenolive in einem Übergangsbereich zwischen der Anlagefläche und einer Oberseite des Hauptkörpers vorgesehen.

Besonders vorteilhaft ist es, wenn die Nasenolive bündig in eine Oberseite des Hauptkörpers übergeht. Hierzu ist eine stetige stufen- und kantenfrei Fläche vorgesehen, die sich von der Oberseite des Hauptkörpers bis zu einer Oberseite der Nasenolive erstreckt. Durch die bündige Ausgestaltung ist es insbesondere in vorteilhafter Art und Weise möglich, die Austragvorrichtung zunächst von unten an die Nase heranzuführen, bis ein Nasenflügel der Nase durch die Nasenolive leicht angehoben wird. Anschließend kann die Austragvorrichtung in ihrer Gesamtheit weitgehend horizontal auf den Benutzer zugerückt werden, wobei die Nasenolive in das dem Nasenflügel zugeordnete Nasenloch einrückt und die Oberseite des Hauptkörpers den Nabenflügel in seiner angehobenen Stellung stützt.

Die Nasenolive kann derart gestaltet sein, dass ihre Haupterstreckungsrichtung und die Austragrichtung sich unterscheiden, beispielsweise um bis zu 90° voneinander abweichen. Als besonders vorteilhaft wird es jedoch angesehen, dass eine Haupterstreckungsrichtung der Nasenolive mit einer durch die Geometrie der Austragöffnung definierten Austragrichtung lediglich einen Winkel zwischen 0° und 30° einschließt. Vorzugsweise beträgt dieser Winkel 0°, so dass die Austragrichtung mit der Haupterstreckungsrichtung der Nasenolive übereinstimmt.

Als besonders vorteilhaft wird es angesehen, wenn eine Austragvorrichtung des beschriebenen Typs eine Betätigungshandhabe aufweist, die an einer Oberseite des Hauptkörpers angeordnet ist und/oder gegenüber dem Hauptkörper in einer Betätigungsrichtung relativ beweglich ist, wobei die Betätigungsrichtung mit einer Haupterstreckungsrichtung der Nasenolive einen Winkel zwischen 75° und 90° einschließt.

Die Gestaltung mit der Betätigungshandhabe an der Oberseite des Hauptkörpers erlaubt eine bequeme Betätigung mit Zeigefinger oder Daumen. Die Betätigungshandhabe ist dabei vorzugsweise derart angeordnet, dass die Betätigung durch die Nase selbst nicht eingeschränkt wird. Vorzugsweise ist die Betätigungshandhabe hierzu zumindest so angeordnet oder derart groß ausgebildet, dass in einem Abstand von etwa 20 mm von der Anlagefläche, die zu dem Zeitpunkt der Betätigung im Bereich der Oberlippe am Mund anliegt, eine Betätigung möglich bleibt. Vorzugsweise ist die Betätigungshandhabe auf der der Anlagefläche abgewandten Seite der Oberseite angeordnet. Die Anordnung an der Oberseite des Hauptkörpers hat den Vorteil, dass eine falsche Ausrichtung der Austragvorrichtung erschwert wird. Wenn ein Benutzer die Austragvorrichtung derart hält, dass die weitgehend horizontal ausgebildete Nasenolive durch Drehen der Austragvorrichtung um 180° fälschlicherweise weitgehend vertikal ausgerichtet wird, so liegt die Betätigungshandhabe in Folge dessen im Bereich seines Mundes oder seines Kinns und kann dementsprechend nicht ohne weiteres betätigt werden. Die falsche Haltung der Austragvorrichtung wird dem Benutzer dadurch offensichtlich. Durch die Gestaltung der Betätigungshandhabe, gemäß der eine Betätigungsrichtung mit der Haupterstreckungsrichtung der Nasenolive einen Winkel von 75° bis 90° einschließt, führt zu einer besonders angenehmen Betätigung, da die Betätigung keine oder nur eine geringfügige Auswirkung auf die Position der Nasenolive innerhalb der Nase des Benutzers hat. Da die Kraftbeaufschlagung der Betätigungshandhabe und die entsprechende Gegenbeaufschlagung des Hauptkörpers nahezu orthogonal zur Einführrichtung der Nasenolive erfolgt, wird die Nasenolive durch die Kraftbeaufschlagung weder weiter in die Nase eingeschoben noch aus der Nase herausgezogen.

Was den Aufbau des Hauptkörpers angeht, so wird es als besonders vorteilhaft angesehen, wenn eine Pumpeinrichtung vorgesehen ist, deren Pumprichtung mit der Vertikalen einen Winkel zwischen 0° und 10° einschließt. Hierdurch kann eine in etwa in Richtung der Vertikalen erfolgenden Betätigung der Betätigungseinrichtung ohne weitere Umlenkmittel unmittelbar zur Betätigung der Pumpeinrichtung genutzt werden.

Als besonders vorteilhaft wird es weiterhin angesehen, wenn im Hauptkörper ein separat handhabbarer Pumpspender vorgesehen ist, der einen Medienspeicher und eine Pumpeinrichtung umfasst. Ein solch separat handhabbarer Pumpspender ist besonders preisgünstig herzustellen und erlaubt eine besonders einfache Gestaltung der übrigen Komponenten der Austragvorrichtung, da die eher komplexen Komponenten der Austragvorrichtung wie der Medienspeicher und die Pumpeinrichtung bereits im separat handhabbaren Pumpspender vorgesehen sind. Die Pumpeinrichtung des Pumpspenders ist vorzugsweise mit einer formflexiblen Leitung wie einem Kunststoffschlauch mit der Austragöffnung verbunden.

Bei einer Weiterbildung der Erfindung weist die Anlagefläche eine Formgebung aufweist, deren Form an die Form des Lippen- und/oder Kinnbereichs eines Benutzers angepasst ist. Hierdurch ist für den Benutzer unmittelbar ersichtlich, wie er die Austragvorrichtung bestimmungsgemäß zu halten hat. Die Formgebung der Anlagefläche und die Erhebung der Nasenolive über diese Anlagefläche kann so geartet sein, dass die Anlagefläche für eine flächige Anlage an den Lippen und/oder dem Kinn des Benutzers ausgebildet ist. Es ist jedoch auch bei einer Ausgestaltung der Austragvorrichtung, bei der die Anlagefläche bestimmungsgemäß in geringem Abstand zu den Lippen und/oder dem Kinn verbleibt, zweckmäßig, eine solche Form der Anlagefläche vorzusehen, um die gewünschte Information über das korrekte Halten der Austragvorrichtung für den Benutzer ersichtlich zu machen.

### Kurzbeschreibung der Zeichnungen

Weitere Merkmale und Vorteile der Erfindung ergeben sich außer aus den Ansprüchen auch aus einer Beschreibung zweier bevorzugter Ausführungsbeispiele der Erfindung, welche nachfolgend beschrieben sind. Dabei zeigen:
- Figur 1 und Figur 2: eine erfindungsgemäße Austragsvorrichtung in einer geschnittenen Seitendarstellung sowie ei- ner nicht geschnittenen Frontdarstellung,
- Figur 3: die Austragvorrichtung der Figuren 1 und 2 in einer perspektivischen Ansicht,
- Figur 4a bis 4c: die korrekte Anwendung der Austragvorrichtung der Figuren 1 bis 3,
- Figur 5a und 5b: falsche Anwendungen der Austragvorrichtung der Figuren 1 bis 3 und
- Figur 6: eine alternative Ausgestaltung der Austragvor- richtung.

### Detaillierte Beschreibung der Ausführungsbeispiele

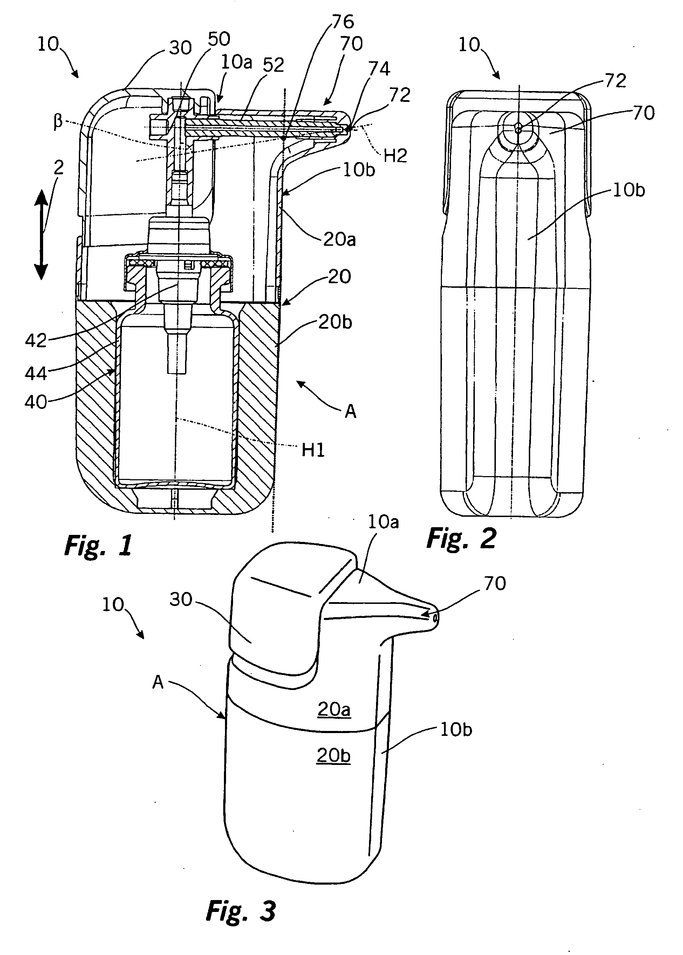

Die Figuren 1 und 2 zeigen eine erfindungsgemäße Austragvorrichtung zur nasalen Applikation von Medikamenten in einer Seitendarstellung und einer Frontdarstellung. Die Figur 3 zeigt die Austragvorrichtung in einer perspektivischen Darstellung. Die dargestellte Austragvorrichtung 10 weist ein zweiteiliges Gehäuse 20 auf, welches aus miteinander verrasteten Gehäusehälften 20a, 20b besteht. Weiterhin ist an einer Oberseite 10a des Gehäuses 20 eine Betätigungshandhabe 30 vorgesehen, die in einer vertikalen Richtung 2 gegenüber dem Gehäuse 20 bewegbar ist.

Auf einer in Fig. 1 rechten Seite wird das Gehäuse durch eine Anlagefläche 10b abgeschlossen, die in der Schnittebene der Fig. 1 weitgehend eben ausgebildet ist. Zwischen dieser Anlagefläche 10b und der Oberseite 10a ist eine Nasenolive 70 einstückig an die obere Gehäusehälfte 20a angeformt.

In das Gehäuse 20 eingesetzt ist ein Pumpspender 40 bestehend aus einer Pumpeinrichtung 42 und einem Medienspeicher 44, auf den die Pumpeinrichtung 42 aufgesetzt ist. Zwischen der Betätigungshandhabe 30 und der Pumpeinrichtung 42 des Pumpspenders 40 ist eine L-förmiger Kanalabschnitt 50 eingesetzt, der eine auf die Betätigungshandhabe 30 in vertikaler Richtung 2 aufgebrachte Kraft auf die Pumpeinrichtung 42 überträgt und dadurch die Betätigung der Pumpeinrichtung 42 mittels der Betätigungshandhabe 30 ermöglicht. Der L-förmige Kanalabschnitt ist dabei derart auf die Pumpeinrichtung 42 aufgesetzt, dass das während eines Pumpvorgangs geförderte Medium in den L-förmigen Kanalabschnitt 50 hineingedrückt wird. Am gegenüberliegenden Ende des L-förmigen Kanalabschnitts 50 schließt sich ein Kunststoffschlauch 52 an, der zur Weiterleitung des Mediums zu einer Austragöffnung 72 am distalen Ende der Nasenolive 70 vorgesehen ist.

Die Formgebung der Austragvorrichtung ist folgendermaßen gestaltet. Der überwiegende Teil des Gehäuse 20 sowie die Betätigungshandhabe 30 sind Teil eines Hauptkörpers A. Dieser Hauptkörper A weist eine insgesamt schlanke Gestalt auf, die sich in Richtung einer Haupterstreckungsrichtung H1 erstreckt. An seinem oberen Ende wird der Hauptkörper A dadurch die Oberseite 10a des Gehäuses 10 und an seiner in Fig. 1 rechten Seite, die während der Verwendung auf den Benutzer zu weist, durch die Anlagefläche 10b abgeschlossen. Die Anlagefläche 10b erstreckt sich nahezu über die vollständige Länge der Austragvorrichtung in vertikale Richtung und ist nahezu eben. Wie bereits beschrieben ist in einen Übergangsbereich zwischen dieser Anlagefläche 10b und der Oberseite 10a die Nasenolive 70 angeordnet. Diese Nasenolive 70 ist gegenüber der Anlagefläche 10b erhaben und ragt also über die durch die Anlagefläche 10b gebildete Ebene beziehungsweise eine dazu fluchtende Ebene, die in der Fig. 1 gepunktet dargestellt ist, hinaus. Sie erstreckt sich dabei in einer Haupterstreckungsrichtung H2, welche in etwa der Einführungsrichtung der Nasenolive 70 entspricht und welche durch eine Verbindungslinie zwischen einem distalen Ende 74 und dem Mittelpunkt 76 der Querschnittsfläche in der Ebene der Anlagefläche 10b entspricht.

Die Betätigung der Austragvorrichtung 10 mittels der Betätigungshandhabe 30 erfolgt in Richtung der Haupterstreckungsrichtung H1. Die Haupterstreckungsrichtung H1 des Hauptkörpers A und die Haupterstreckungsrichtung H2 der Nasenolive 70 schließen einen Winkel β von etwa 80° ein.

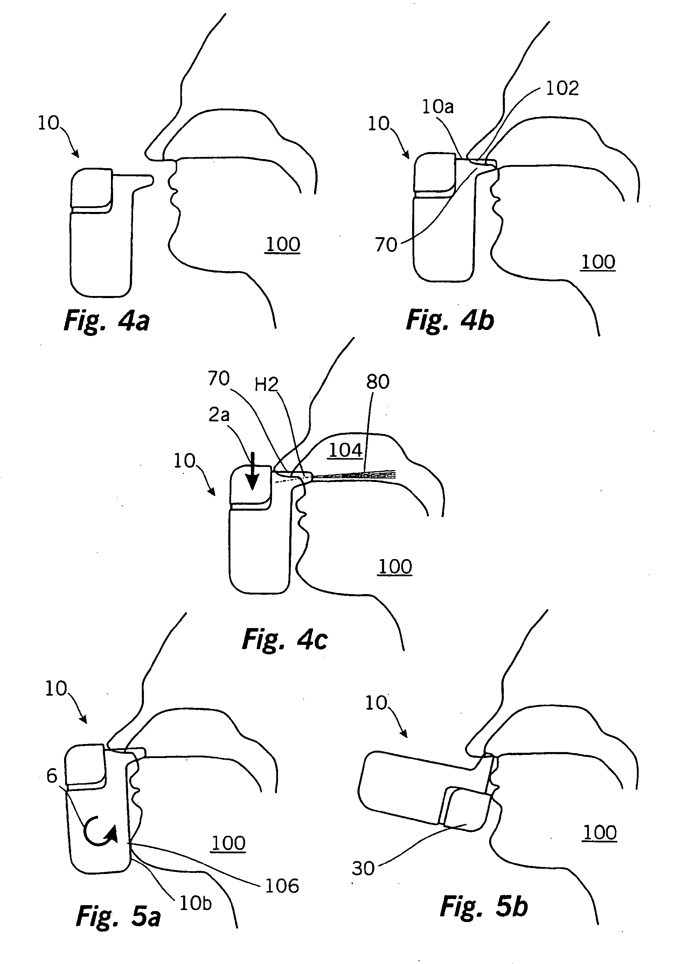

Die Figuren 4a bis 4c zeigen die korrekte Verwendung der Austragvorrichtung.

Dabei verdeutlicht die Figur 4a zunächst nur die Ausrichtung der Austragvorrichtung. Wie zu ersehen ist, wird die Austragvorrichtung 10 in der Ausrichtung, in der sie auch in den Figuren 1 und 2 dargestellt ist, an den Mund- und Nasenbereich eines aufrecht sitzenden oder stehenden Patienten 100 herangeführt. Mittels der Oberseite 10a wird dann - wie in Figur 4b dargestellt ist - ein Nasenflügel 102 des Benutzers leicht angehoben. Anschließend wird, wie in Figur 4c dargestellt ist, die Austragvorrichtung in ihrer Gesamtheit näher an den Benutzer herangedrückt, wobei die Nasenolive 70 bis in den Eingangsbereich der Nasenhöhle 104 gelangt. In diesem in Figur 4c dargestellten Zustand erfolgt dann die Betätigung durch Herunterdrücken der Betätigungshandhabe 30 in Richtung 2a. Dies führt zu einem Sprühstrahl 80, der bis tief in die Nasenhöhle 104 gelangt. Da die Betätigungsrichtung 2a nahezu einen rechten Winkel mit der Haupterstreckungsrichtung H2 der Nasenolive 70 einschließt, wird eine Verschiebung der Nasenolive 70 durch die Betätigung üblicherweise nicht erzielt. Ein dem Benutzer unangenehmes Gefühl durch versehentliches tieferes Einschieben oder versehentliches Herausziehen der Nasenolive 70 im Zuge der Betätigung ist daher nicht zu befürchten.

Die Ausrichtung der Nasenolive 70 verbunden mit deren Sprührichtung, die in etwa der Haupterstreckungsrichtung H2 entspricht führt zu einem besonders wirksamen Eintreten des ausgetragenen Mediums 80 in die Nasenhöhle 104. Die häufig falsche Anwendung von Nasensprays, die insbesondere in vertikaler Richtung austragend eingesetzt werden, wird verhindert.

Eine falsche Verwendung durch Verkippen der Austragvorrichtung 10 in Richtung des Pfeils 6 wie in Fig. 5a dargestellt ist, ist nicht möglich, da die Anlagefläche 10b der Austragvorrichtung 10 hierbei am Mund oder dem Kinn 106 des Benutzers anstoßen würde. Ein vollständiges Verschwenken, so dass die Austragvorrichtung wie in 5b dargestellt verwendet wird, ist jedoch auch nicht zu befürchten, da aufgrund der schlanken Form der Austragvorrichtung 10 die Stellung der Fig. 5b dem Benutzer zu Recht ungewöhnlich vorkäme. Zudem wäre die Betätigungshandhabe 30 in dieser Stellung kaum zu bedienen, so dass offensichtlich ist, dass die in Figur 5b dargestellte Art der Benutzung nicht gewollt sein kann.

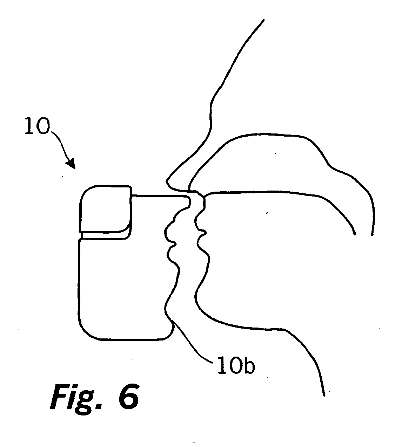

Fig. 6 zeigt eine alternative Ausgestaltung einer erfindungsgemäßen Austragvorrichtung. Diese zweite Austragvorrichtung 10 entspricht bezüglich ihrem Aufbau der oben beschriebenen ersten Ausgestaltung, unterscheidet sich jedoch dadurch dass, die Anlagefläche eine Formgebung aufweist, deren Form an die Form des Lippen- und Kinnbereichs eines Benutzers angepasst ist. Die bestimmungsgemäße Lage der Austragvorrichtung bei der Verwendung ist dadurch für den Benutzer unmittelbar ersichtlich.

## Patentansprüche

1. **Nasenspray-Vorrichtung** (10) zur nasalen Applikation von Medikamenten durch Ausbringung eines Sprühstrahls mit
- einem Hauptkörper (A), der eine bei Benutzung dem Gesicht des Benutzers zugewandte und vertikal ausgerichtete Anlagefläche (10b) aufweist, sowie
- einer ortsfest zum Hauptkörper (A) angeordnete Nasenolive (70) zur Einführung in ein Nasenloch des Benutzers und zur Erzeugung des Sprühstrahls, wobei die Nasenolive (70) an einem oberen Randbereich der Anlagefläche (10b) vorgesehen ist, sich über die Anlagefläche (10b) erhebt und eine Austragöffnung (72) aufweist, wobei
- eine Betätigungshandhabe (30) zur Erzeugung des Sprühstrahls vorgesehen ist, die an einer sich an die Nasenolive (70) anschließenden Oberseite (10a) des Hauptkörper (A) angeordnet ist und gegenüber dem Hauptkörper (A) in einer Betätigungsrichtung (2) relativbeweglich ist, **dadurch gekennzeichnet**, daβ die Betätigungsrichtung (2) mit einer Haupterstreckungsrichtung (H2) der Nasenolive (70) einen Winkel (β) zwischen 75° und 90° einschließt.

2. Nasenspray-Vorrichtung nach Anspruch 1,
**dadurch gekennzeichnet, dass**
die vertikale Anlagefläche (10b) eine Höhe von mindestens 40mm, vorzugsweise von mindestens 60mm aufweist.

3. **Nasenspray-Vorrichtung** nach Anspruch 1 oder 2,
**dadurch gekennzeichnet, dass**
die Nasenolive (70) sich um mindestens 10mm über die Anlagefläche (10b) erhebt.

4. **Nasenspray-Vorrichtung** nach einem der vorstehenden Ansprüche,
**dadurch gekennzeichnet, dass**
eine Haupterstreckungsrichtung (H1) des Hauptkörpers (A) sich parallel zur vertikal ausgerichteten Anlagefläche (10b) erstreckt.

5. **Nasenspray-Vorrichtung** nach einem der vorstehenden Ansprüche,
**dadurch gekennzeichnet, dass**
eine Haupterstreckungsrichtung (H2) der Nasenolive (70) mit einer Vertikalen (2) einen Winkel zwischen 45° und 90° einschließt, vorzugsweise zwischen 60° und 90°.

6. **Nasenspray-Vorrichtung** nach einem der vorstehenden Ansprüche,
**dadurch gekennzeichnet, dass**
die Nasenolive (70) bündig in eine Oberseite (10a) des Hauptkörpers (A) übergeht.

7. **Nasenspray-Vorrichtung** nach einem der vorstehenden Ansprüche,
**dadurch gekennzeichnet, dass**
eine Haupterstreckungsrichtung (H2) der Nasenolive (70) mit einer durch die Geometrie der Austragöffnung (72) definierten Austragrichtung einen Winkel zwischen 0° und 30° einschließt.

8. **Nasenspray-Vorrichtung** nach einem der vorstehenden Ansprüche,
**dadurch gekennzeichnet, dass**
im Hauptkörper (A) eine Pumpeinrichtung (42) vorgesehen ist, deren Pumprichtung mit der Vertikalen einen Winkel zwischen 0° und 10° einschließt.

9. **Nasenspray-Vorrichtung** nach einem der vorstehenden Ansprüche,
**dadurch gekennzeichnet, dass**
im Hauptkörper (A) ein separat handhabbarer Pumpspender (40) vorgesehen ist, der einen Medienspeicher (44) und eine Pumpeinrichtung (42) umfasst.

10. **Nasenspray-Vorrichtung** nach einem der vorstehenden Ansprüche,
**dadurch gekennzeichnet, dass**
die Anlagefläche (10b) eine Formgebung aufweist, deren Form an die Form des Lippen- und/oder Kinnbereichs eines Benutzers angepasst ist.

## Claims

1. Nasal spray device (10) for nasal application of medicines by discharge of a spray jet
with
- a main element (A) having a vertically aligned contact surface (10b) facing the face of the user during use and
- a nose olive (70) arranged stationarily to the main element (A) for insertion into a nostril of the user and for generation of a spray jet, said nose olive (70) being provided on an upper rim area of the contact surface (10b), projecting above said contact surface (10b) and having a discharge opening (72), where
- an actuating handle (30) being provided for generating the spray jet, being arranged on an upper side (10a) of the main element (A) adjoining the nose olive (70) and being movable relative to said main element (A) in an actuation direction (2),
**characterized in that**
the actuation direction (2) describes with a main extension direction (H2) of the nose olive (70) an angle (ß) between 75° and 90°.

2. Nasal spray device according to Claim 1,
**characterized in that**
the vertical contact surface (10b) has a height of at least 40 mm, preferably at least 60 mm.

3. Nasal spray device according to Claim 1 or 2,
**characterized in that**
the nose olive (70) projects at least 10 mm above the contact surface (10b).

4. Nasal spray device according to one of the preceding claims,
**characterized in that**
the main extension direction (H1) of the main element (A) extends parallel to the vertically aligned contact surface (10b).

5. Nasal spray device according to one of the preceding claims,
**characterized in that**
a main extension direction (H2) of the nose olive (70) describes with a vertical (2) an angle between 45° and 90°, preferably between 60° and 90°.

6. Nasal spray device according to one of the preceding claims,
**characterized in that**
the nose olive (70) merges flush into an upper side (10a) of the main element (A).

7. Nasal spray device according to one of the preceding claims,
**characterized in that**
a main extension direction (H2) of the nose olive (70) describes with a discharge direction defined by the geometry of the discharge opening (72) an angle between 0° and 30°.

8. Nasal spray device according to one of the preceding claims,
**characterized in that**
inside the main element (A) a pump device (42) is provided whose pump direction describes with the vertical an angle between 0° and 10°.

9. Nasal spray device according to one of the preceding claims,
**characterized in that**
inside the main element (A) a separately operable pump dispenser (40) is provided comprising a media reservoir (44) and a pump device (42).

10. Nasal spray device according to one of the preceding claims,
**characterized in that**
the contact surface (10b) has been shaped such that its shape is adapted to the shape of the lip area and/or chin area of a user.

## Revendications

1. Dispositif de pulvérisation nasale (10) destiné à l'application nasale de médicaments par décharge d'un jet pulvérisé avec
- un corps principal (A), qui présente une surface d'appui (10b) orientée à la verticale et tournée vers le visage de l'utilisateur lors de l'utilisation, ainsi que
- une olive nasale (70), disposée de manière fixe par rapport au corps principal (A), destinée à être introduite dans une narine de l'utilisateur et à produire le jet pulvérisé, l'olive nasale (70) étant prévue sur une zone de bord supérieure de la surface d'appui (10b), s'élevant au-dessus de la surface d'appui (10b) et présentant une ouverture de distribution (72), sachant que
- une manette d'actionnement (30) est prévue pour la production du jet pulvérisé, laquelle manette est disposée sur une face supérieure (10a) du corps principal (A) prolongeant l'olive nasale (70) et peut être déplacée par rapport au corps principal (A) dans une direction d'actionnement (2), **caractérisé en ce que** la direction d'actionnement (2) inclut avec une direction d'extension principale (H2) de l'olive nasale (70) un angle (β) compris entre 75° et 90°.

2. Dispositif de pulvérisation nasale selon la revendication 1,
**caractérisé en ce**
**que** la surface d'appui verticale (10b) présente une hauteur d'au moins 40 mm, de préférence d'au moins 60 mm.

3. Dispositif de pulvérisation nasale selon la revendication 1 ou 2,
**caractérisé en ce**
**que** l'olive nasale (70) s'élève d'au moins 10 mm au-dessus de la surface d'appui (10b).

4. Dispositif de pulvérisation nasale selon l'une des revendications précédentes,
**caractérisé en ce**
**qu'**une direction d'extension principale (H1) du corps principal (A) s'étend parallèlement à la surface d'appui (10b) orientée verticalement.

5. Dispositif de pulvérisation nasale selon l'une des revendications précédentes,
**caractérisé en ce**
**qu'**une direction d'extension principale (H2) de l'olive nasale (70) inclut avec une verticale (2) un angle compris entre 45° et 90°, de préférence entre 60° et 90°.

6. Dispositif de pulvérisation nasale selon l'une des revendications précédentes,
**caractérisé en ce**
l'olive nasale (70) se prolonge de niveau en une face supérieure (10a) du corps principal (A).

7. Dispositif de pulvérisation nasale selon l'une des revendications précédentes,
**caractérisé en ce**
**qu'**une direction d'extension principale (H2) de l'olive nasale (70) inclut avec une direction de distribution définie par la géométrie de l'ouverture de distribution (72) un angle compris entre 0° et 30°.

8. Dispositif de pulvérisation nasale selon l'une des revendications précédentes,
**caractérisé en ce**
**qu'**est prévu dans le corps principal (A) un dispositif de pompage (42) dont la direction de pompage inclut avec la verticale un angle compris entre 0° et 10°.

9. Dispositif de pulvérisation nasale selon l'une des revendications précédentes,
**caractérisé en ce**
**qu'**est prévu dans le corps principal (A) un distributeur à pompe (40) maniable séparément qui comprend un réservoir de produit (44) et un dispositif de pompage (42).

10. Dispositif de pulvérisation nasale selon l'une des revendications précédentes,
**caractérisé en ce**
**que** la surface d'appui (10b) présente une formation dont la forme est adaptée à la forme de la zone labiale et/ou du mentonnière d'un utilisateur.
